# EUROPEAN PATENT APPLICATION

(11) **EP 3 831 944 A1**
(43) Date of publication of application: **09.06.2021**
(21) Application number: 19845524.8
(22) Date of filing: 26.07.2019
(51) Int. Cl.: C12N 15/13, A61K 38/08, A61K 39/395, A61K 47/68, A61P 35/00, C07K 16/28, C07K 16/46, C12P 21/08

(54) **ANTI-ROR1 MONOCLONAL ANTIBODY, FUNCTIONAL FRAGMENT THEREOF, GENE, DRUG DELIVERY COMPOSITION, AND PHARMACEUTICAL COMPOSITION**

(30) Priority: 01.08.2018 JP 2018145061
(71) Applicant: National University Corporation Tokai National Higher Education and Research System, Nagoya-shi Aichi 464-8601 (JP)
(72) Inventor: TAKAHASHI Takashi, Aichi 4648601 (JP)
(74) Representative: Heyerhoff Geiger & Partner Patentanwälte PartGmbB
(86) International application number: PCT/JP2019/029481
(87) International publication number: WO 2020/026987

(57) **Abstract**

The present invention addresses the problem of providing an anti-ROR1 monoclonal antibody that is taken in a cancer cell with ROR1 during endocytosis of the cancer cell. This problem can be solved by an anti-ROR1 monoclonal antibody that recognizes the amino acid sequence from position 156 to 300 of human ROR1 protein (GenBank accession No. NP_005003.2) as an epitope.

## Description

### [Technical Field]

The disclosure in the present specification relates to an anti-ROR1 monoclonal antibody and a functional fragment thereof, a gene, a drug delivery composition, and a pharmaceutical composition. In particular, the disclosure relates to an anti-ROR1 monoclonal antibody and a functional fragment thereof endocytosed in a cancer cell together with an ROR1 during cancer cell endocytosis, a gene having a gene that encodes a variable region of an anti-ROR1 monoclonal antibody as an open reading frame region, a drug delivery composition containing the anti-ROR1 monoclonal antibody or the functional fragment thereof, and a pharmaceutical composition.

### [Background Art]

Lung cancer is a primary cause of death of cancer in economically well-developed countries, and among the lung cancer, lung adenocarcinoma is the most frequent histologic subtype.

It is known that lung adenocarcinoma is closely linked to continuous expression of a thyroid transcription factor-1 (TTF-1) that is a lineage-specific transcription factor required for branching morphogenesis formation and a physiological function in a lung (see Non-Patent Literature 1). Further, it is also known that the TTF-1 is a lineage-survival oncogene (see Non-Patent Literatures 2 to 4). Since the TTF-1 is indispensable for production of a surfactant protein and a physiological function in a lung of a healthy adult, identification of a downstream molecule involved in a TTF-1 lineage-specific survival signal is required for development of a novel treatment method.

As a downstream molecule induced to express by the TTF-1, a receptor tyrosine kinase-like orphan receptor 1 (ROR1) gene is known. Further, it is also known that it is possible to inhibits proliferation of particular cancer cells by suppressing expression of such an ROR1 gene (see Patent Literature 1).

Furthermore, it is known that targeting ROR1 enables not only induction of apoptosis of a cancer cell but also suppression of a pro-survival signal of a cancer cell transferred by receptor tyrosine kinase (hereafter, receptor tyrosine kinase may be referred to as "RTK") such as EGFR or MET and therefore the ROR1 can be an important target in development of a proliferation inhibitor against a cancer cell (see Patent Literature 2 and Non-Patent Literature 5).

As disclosed in Patent Literature 2 or the like described above, most of molecular targeted drugs that have been developed so far are kinase activation inhibitors. The kinase activation inhibitor refers to a compound that suppresses activation of RTK related to proliferation and survival of human cancer such as lung cancer. If a kinase activation inhibitor is administered to a patient, however, this may cause mutation that provides resistance to a kinase gene during therapy or occurrence of a bypass path by which a survival signal of a cancer cell depends on another receptor, which causes a problem of cancer cells gaining resistance against the kinase activation inhibitor.

Further, when one receptor of a cancer cell, such as EGFR, MET, or the like disclosed in Patent Literature 2 described above is a molecule target, a sufficient effect may not be obtained because a signal from another receptor is unable to be blocked, and combined use of inhibitors targeting respective receptors have been attempted. Even with combined use of a plurality of inhibitors, however, there is a problem of likelihood of further occurrence of a bypass path or an increased side effect due to administration of the plurality of inhibitors.

For the above reasons, a normal cell or a cancer cell enables itself to survive by effectively using various survival signals, and these survival signals are transferred via many pieces of RTK existing in cell membranes. From the past studies, molecular targeted drugs (such as Iressa targeting EGFR) targeting individual RTK have been developed against cancer cells, and combined use of inhibitors against multiple types of RTK as described above has been attempted. However, since occurrence of a bypass path via further different RTK or these types of RTK have an important role of transferring a survival signal also in a normal cell, a side effect is considered to be a significantly important problem. Thus, it is known that, by suppressing coupling of ROR1 and Cavin-1 or ROR1 and Caveolin1 (hereafter, which may be referred to as "CAV1") rather than targeting individual RTK of a cancer cell, it is possible to suppress formation of caveola in which various proliferation factors are coupled and RTK that transfers a signal into a cell is integrated and it is possible to eliminate the activity of various types of RTK by specifically suppressing formation of caveola (see Patent Literature 3 and Non-Patent Literature 6).

### [Citation List]

### [Patent Literature]

Patent Literature 1: International Publication No. 2010/008069
Patent Literature 2: International Publication No. 2012/090939
Patent Literature 3: International Publication No. 2016/063637

### [Non-Patent Literature]

Non-Patent Literature 1: Tanaka.H et al.,Cancer Res,2007,Vol.67, p6007-6011
Non-Patent Literature 2: Kendall.J et al., Proc Natl Acad Sci USA,2007, Vol.104, p16663-16668
Non-Patent Literature 3: Weir,BA et al., Nature, 2007, Vol.450, p893-898
Non-Patent Literature 4: Kwei,KA et al., Oncogene,2008, Vol.27, p3635-3640
Non-Patent Literature 5: Yamaguchi.T et al., Cancer Cell,2012, Vol.21, p348-361
Non-Patent Literature 6: Yamaguchi.T et al., Nature Commun, 2016, Vol. 7,10060 (doi:10.1038/ncomms10060)

### [Summary of Invention]

### [Technical Problem]

As described above, the invention disclosed in Patent Literature 3 is very effective in searching for a compound that can eliminate activity of various types of RTK by specifically suppressing formation of caveola. In this regard, a cell enables itself to survive by effectively using various survival signals. Thus, to develop an effective therapeutic agent against cancer, it is necessary to focus on various molecular mechanisms of cancer cells and search for an effective method.

The disclosure in the present specification is the invention that has been made in order to solve the conventional problems described above, and according to a thorough study, it has been newly found that (1) when an ROR1 that is a protein unique to a cancer cell is focused on, an anti-ROR1 antibody that recognizes a specific region of an extracellular region of the ROR1 as an epitope is endocytosed in a cancer cell together with the ROR1 during endocytosis and (2) thus, an anti-ROR1 antibody can be used as a delivery that endocytoses a drug in a cell.

That is, the object of the disclosure in the present specification is to provide an anti-ROR1 monoclonal antibody and a functional fragment thereof endocytosed in a cancer cell together with an ROR1 during cancer cell endocytosis, a gene having a gene that encodes a variable region of an anti-ROR1 monoclonal antibody as an open reading frame region, a drug delivery composition containing the anti-ROR1 monoclonal antibody or the functional fragment thereof, and a pharmaceutical composition.

### [Solution to Problem]

The disclosure in the present specification relates to an anti-ROR1 monoclonal antibody and a functional fragment thereof, a gene, a drug delivery composition, and a pharmaceutical composition described below.

(1) An anti-ROR1 monoclonal antibody that recognizes positions 156 to 300 in the amino acid sequence of a human ROR1 protein (GenBank accession No. NP_005003.2) as an epitope.
(2) The anti-ROR1 monoclonal antibody according to (1) above, wherein the anti-ROR1 monoclonal antibody is specifically coupled to an ROR1 of a human cancer cell and endocytosed in a cell by endocytosis.
(3) The anti-ROR1 monoclonal antibody according to (1) or (2) above,
   wherein a heavy-chain variable region includes a region consisting of
   a CDR1 H region consisting of an amino acid sequence of SYGIS (SEQ ID No. 18),
   a CDR2 H region consisting of an amino acid sequence of EISPRSGYTYYNEKFKG (SEQ ID No. 19), and
   a CDR3 H region consisting of an amino acid sequence of PHYGDYVGY (SEQ ID No. 20), and
   wherein a light-chain variable region includes a region consisting of
   a CDR1 L region consisting of an amino acid sequence of RSSQSLVHSNGNTYLH (SEQ ID No. 23),
   a CDR2 L region consisting of an amino acid sequence of KVSNRFS (SEQ ID No. 24), and
   a CDR3 L region consisting of an amino acid sequence of SQSTHVPFT (SEQ ID No. 25).
(4) The anti-ROR1 monoclonal antibody according to any one of (1) to (3) above,
   wherein the amino acid sequence of an H-chain variable region is of SEQ ID No. 17, and
   wherein the amino acid sequence of an L-chain variable region is of SEQ ID No. 22.
(5) A functional fragment of the anti-ROR1 monoclonal antibody according to any one of (1) to (4) above.
(6) A gene having, as an open reading frame region, a gene that encodes the CDR sequence according to (3) above or the variable region according to (4) above.
(7) A drug delivery composition including the anti-ROR1 monoclonal antibody according to any one of (1) to (4) above or the functional fragment of the anti-ROR1 monoclonal antibody according to (5) above.
(8) A pharmaceutical composition including:
   the anti-ROR1 monoclonal antibody according to any one of (1) to (4) above or the functional fragment of the anti-ROR1 monoclonal antibody according to (5) above;
      a linker; and
      a drug,
   wherein one terminal of the linker is coupled to the anti-ROR1 monoclonal antibody or the functional fragment, and the other terminal of the linker is coupled to the drug.

### [Advantageous Effect of Invention]

The anti-ROR1 monoclonal antibody disclosed in the present specification is endocytosed in a cancer cell together with an ROR1 that is a protein unique to a cancer cell during cancer cell endocytosis. Thus, the anti-ROR1 monoclonal antibody disclosed in the present specification can be used as a drug delivery into a cancer cell or a pharmaceutical composition.

### [Brief Description of Drawings]

[FIG. 1] FIG. 1 is a diagram illustrating the structure of an ROR1 molecule and a recognition domain of an acquired monoclonal antibody and a diagram illustrating homology of a mouse ROR1 and a human ROR1.
[FIG. 2] FIG. 2A is a diagram illustrating response of anti-human ROR1 antibodies to ROR1 expressing cells of acquired monoclonal antibodies (2AM series and 2AC series). FIG. 2B is a diagram illustrating response of anti-human ROR1 antibodies to ROR1 expressing cells of acquired monoclonal antibodies (2AA series, 2CC series, and 1AA series).
[FIG. 3] FIG. 3C is a diagram illustrating response of anti-human ROR1 antibodies to ROR1 expressing cells of acquired monoclonal antibodies (2DC series and some of 1BA series). FIG. 3D is a diagram illustrating response of anti-human ROR1 antibodies to ROR1 expressing cells of acquired monoclonal antibodies (1BA series).
[FIG. 4] FIG. 4E is a diagram illustrating response of anti-human ROR1 antibodies to ROR1 expressing cells of acquired monoclonal antibodies (6AM series). FIG. 4F is a diagram illustrating response of anti-human ROR1 antibodies to ROR1 expressing cells of acquired monoclonal antibodies (70AF series).
[FIG. 5] FIG. 5A is a diagram illustrating the names of origin clones of chimerized antibodies and recognition domains. FIG. 5B is a diagram illustrating structure of chimerized antibody and recognition domains.
[FIG. 6] FIG. 6 is a diagram illustrating response of chimerized antibodies to ROR1 high expressing cells and ROR1 low expressing cells.
[FIG. 7] FIG. 7 is a diagram illustrating endocytosis of chimerized antibodies to ROR1 high expressing cells and ROR1 low expressing cells.
[FIG. 8] FIG. 8A is a diagram illustrating cell proliferation inhibition of the PC-9 by a chimerized antibody and a non-cleavage type MMAF. FIG. 8B is a diagram illustrating cell proliferation inhibition of the PC-9 by a chimerized antibody M2 and a cleavage type MMAF. FIG. 8C is a diagram illustrating cell proliferation inhibition of the A427 by a chimerized antibody M2 and a cleavage type MMAF.

### [Description of Embodiments]

An anti-ROR1 monoclonal antibody (hereafter, which may be simply referred to as "antibody") and a functional fragment thereof, a gene, a drug delivery composition, and a pharmaceutical composition will be described below in detail.

The antibody disclosed in the present specification means an antibody that recognizes, as an epitope, positions 156 to 300 in the amino acid sequence of the extracellular region of a human ROR1 and is specifically coupled thereto and that includes a fragment (which may be referred to as a "functional fragment" in the present specification) or a derivative of the antibody exhibiting substantially the same antigen specificity as the original antibody. The functional fragment or the derivative of an antibody includes a functional fragment of the antibody, such as a peptide containing Fab, Fab', F(ab')₂, a single chain antibody (scFv), a disulfide-stabilized V region fragment (dsFv), or CDR, a derivative such as a humanized antibody (for example, a CDR-implant complete human antibody), or the like. The derivative can be created by a known method by using a gene recombination technology.

Amino acid sequences, gene information, and the cDNA sequences of a human ROR1 are well known, and information on the sequence or the like is available from the amino acid sequence deposited in GenBank accession No. NP_005003.2, the gene information on GenBank Gene ID: 4919, and the cDNA deposited in GenBank accession No. NM_005012.1, for example. The full length of the amino acid sequence of the human ROR1 is 937, and positions 1 to 403 in the amino acid sequence correspond to the extracellular region.

Further, in the present specification, when "positions 156 to 300 in the amino acid sequence of the human ROR1 protein (GenBank accession No. NP_005003.2)" is stated, in addition to positions 156 to 300 in the amino acid sequence, the derivatives thereof are included. The term "derivative" as used herein includes one or a plurality of (for example, several (for example, six)) mutated, substituted, deleted, and/or added amino acid residues at positions 156 to 300 in the amino acid sequence and means peptide or polypeptide substantially having the same antigenicity as those of positions 156 to 300 in the amino acid sequence of the human ROR1. The term "substantially" as used herein means that an antibody created from positions 156 to 300 in the amino acid sequence as an epitope is specifically coupled to an ROR1 and has a function of being endocytosed in a cancer cell together with the ROR1 during endocytosis.

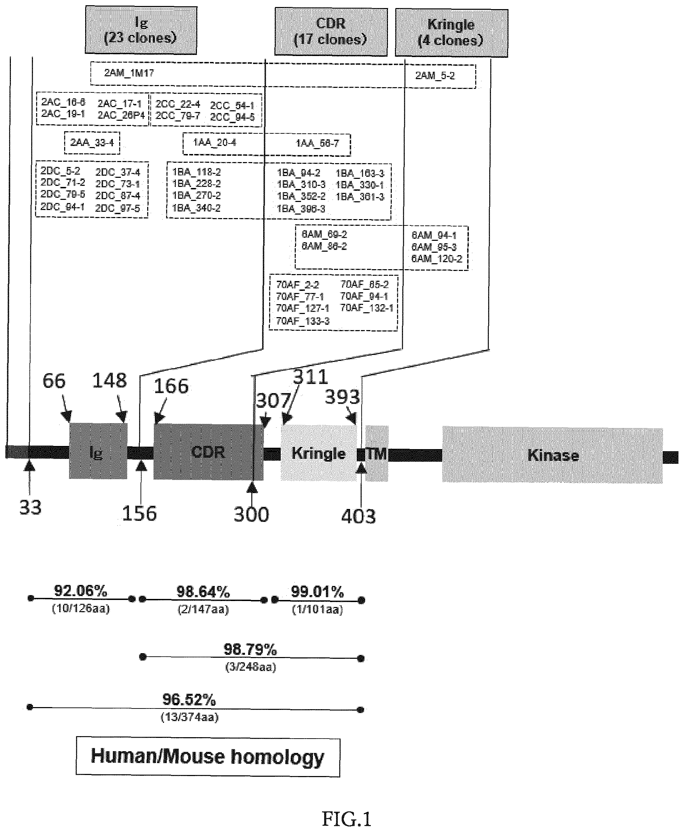

The term "epitope" refers to a part of an antigen recognized by an antibody (in the case of the present specification, positions 156 to 300 in the amino acid sequence of the extracellular region of a human ROR1) and means a site on an antigen to which a region including an antibody variable region disclosed in the present specification is coupled. For example, when polypeptide such as an ROR1 is an antigen, the antibody may recognize a linear amino acid sequence and may recognize a three-dimensional spatial structure, and therefore, the epitope can be defined by an amino acid sequence or antigen structure. Note that the extracellular region of the amino acid sequence of the human ROR1 includes three regions referred to as Ig (amino acid positions: 66 to 148), cysteine-rich domain (CRD, amino acid positions: 166 to 307), and Kringle (amino acid positions: 311 to 393) (see FIG. 1). Therefore, an antibody disclosed in the present specification can be said to be an antibody that substantially recognizes the CRD as an epitope.

The antibody disclosed in the present specification is specifically coupled to an ROR1 of a human cancer cell and endocytosed in the cell by endocytosis as illustrated in Examples described later. Thus, the antibody can be used as an antibody for delivering a drug or the like that damage a cancer cell. Therefore, a composition in which the antibodies disclosed in the present specification are dispersed in a medium or the like can be provided as a drug delivery composition.

Further, it is possible to provide a pharmaceutical composition using the antibody disclosed in the present specification by coupling one end of a linker to the antibody disclosed in the present specification and coupling a drug to the other end of the linker. Any of cleavage type linkers and non-cleavage type linkers may be used for the linker. Further, the drug is not particularly limited as long as it is a drug for cancer therapy such as killing a cancer cell or suppressing proliferation of cancer cells and can be coupled to a linker. Note that, when coupling a linker to an antibody, it is preferable to couple a linker to a site other than the H-chain variable region and the L-chain variable region so as not to inhibit coupling of the antibody to an ROR1.

The cancer cell is not particularly limited as long as an ROR1 has expressed therein and may be a cell of cancer such as, but not limited thereto, lung cancer (including lung adenocarcinoma, small cell lung cancer, squamous cell lung cancer, and large-cell lung cancer), pancreatic cancer, malignant mesothelioma, breast cancer, stomach cancer, colorectal cancer, oral cancer, esophageal cancer, uterine cancer, kidney cancer, bladder cancer, ovarian cancer, testicular cancer, and the like, for example. The biological species from which a cancer cell is derived may be, but not limited to, human, monkey, mouse, rat, guinea pig, pig, cow, sheep, goat, and the like, for example.

While Examples will be presented below and embodiments will be specifically described, these Examples and embodiments are not intended to limit or restrict the scope of the invention disclosed in the present specification.

### [Examples]

### (1) Construction of Various Expression Construct of Human ROR1

A cDNA sequence (NM_005012.1) of the full length of a human ROR1 was cut out from pCMV6-KL6-ROR1 (OriGene Technologies, Rockville, MDs) and introduced in pCMVpuro to create pCMVpuro-ROR1. Furthermore, animal cells that are controlled by a CMV promotor and cause the full length of a human ROR1 (amino acid positions 1 to 937) or the full length of a human ROR1 extracellular region (amino acid positions 1 to 403) to express at the same time as a puromycin-EGFP fusion protein in the downstream of the IRES sequence were obtained by the method below.

### <Full length of human ROR1>

A PCR product amplified by using 5' Primer including a NotI site (SEQ ID No. 1: 5'-AATAGCGGCCGCACCATGCACCGGCCGCGCC-3': the underline indicates the NotI site, and an underline part indicates a site to be cut by a restriction enzyme, though the repeated description will be omitted in the sequences below) and 3' Primer including an XhoI site (SEQ ID No. 2: 5'-GCGGCTCGAGCAGTTCTGCAGAAATCATAGATTCG-3') was digested with restriction enzymes NotI and XhoI, and thereby a fragment was obtained. The obtained fragment was introduced in a NotI-XhoI site of pQCXIPG, and an expression vector pQCXIPG-full was constructed.

### <Full length of human ROR1 extracellular region>

A PCR product obtained by using 5' Primer including a NotI site (SEQ ID No. 1) and 3' Primer including a BamHI site (SEQ ID No. 3: 5'-CCGCGGATCCTTCCATTTTATTCTTCTCCTTGG-3') was digested with NotI and BamHI, and thereby a fragment was obtained. The obtained fragment is introduced in a NotI-BamHI site of pQCXIPG, and an expression vector pQCXIPG-EC was constructed.

On the other hand, a secretory signal sequence of chicken egg white lysozyme (amino acid sequence: MRSLLILVLCFLPLAALG|AAA (SEQ ID No. 46), gene sequence: ATGAGGTCTTTGCTAATCTTGGTGCTTTGCTTCCTGCCCCTGGCTGCTCTGGGG| GCGGCCGCC (SEQ ID No. 47)) was added to the upstream of a multicloning site of the pQCXIPG described above, and a vector (lyssig-pQCXIPG) that enables forced secretion of a translation product of an introduced gene outside the cell was used for a vector used for causing a partial length of a human ROR1 including the human CDR region and the Kringle region (amino acid positions 156 to 403) and a partial length of a human ROR1 including the human CDR region (amino acid positions 156 to 300) to express in an animal cell.

### <Partial length of human ROR1 including human CDR region and Kringle region>

A PCR product amplified by using 5' Primer including a NotI site (SEQ ID No. 4: 5'-AATAGCGGCCGCAAGTCCAGGATACTCAGATGA-3') and 3' Primer including a BamHI site (SEQ ID No. 3) was digested with restriction enzymes NotI and BamHI, and thereby a fragment was obtained. The obtained fragment was introduced in a NotI-BamHI site of lyssig-pQCXIPG, and an expression vector pQCXIPG-CK was constructed. Note that pQCXIPG and lyssig-pQCXIPG are vectors modified based on pQCXIP of BD Retro-XTM Q Vectors.

### <Partial length of human ROR1 including human CDR region>

A PCR product amplified by using 5' Primer (5'-AATAGCGGCCGCAAGTCCAGGATACTCAGATGA-3' (SEQ ID No. 4)) and 3' Primer including a BamHI site (SEQ ID No. 5: 5'-AGCAGGATCCAATCCGGATACAGTTCGCA-3') was digested with restriction enzymes NotI and BamHI, and thereby a fragment was obtained. The obtained fragment was introduced in a NotI-BamHI site of lyssig-pQCXIPG, and an expression vector pQCXIPG-CDR was constructed.

### (2) Establishment of Cell Line Causing Expression of Human ROR1

To establish an antigen expressing cell line, Pantropic Retroviral Expression System (Clontech; K1063-1) was used. A GP2-293 (Clontech; K1063-1) in an 80 to 90% confluent state was provided in a collagen-coated 100 mm dish, and 11.2 µg of an expression vector constructed as described above (pQCXIPG-full, pQCXIPG-EC, pQCXIPG-CK, pQCXIPG-CDR) using Lipofectamine 2000 and 11.2 µg of pVSV-G (Clontech; K1063-1) are co-transfected. After 48 hours, the supernatant containing virus particles was collected, and virus was precipitated by super centrifuge (18,000 rpm, 1,5 hours, 4 degrees Celsius). The precipitate was suspended with 30 µL of TNE (50 mM, Tris-HCl [pH 7.8], 130 mM NaCl, 1mM EDTA), and a retroviral vector concentrated solution was prepared.

Then, 5 µL of the retroviral vector concentrated solution was diluted with 150 µL of DMEM (SIGMA; D5796)-10% FBS containing 8 µg/ml of hexadimethrine bromide (SIGMA; H-9268) to prepare a culture medium containing virus particles. A 293T culture medium provided so as to be in a state of around 40% confluent in a 96-well microplate was replaced with the prepared culture medium containing the virus particles, and thereby pQCXIPG-full, pQCXIPG-EC, pQCXIPG-CK, and pQCXIPG-CDR were genetically introduced. After the genetic introduction, the resultant was amplified and cultured with DMEM (SIGMA; D5796)-10% FBS containing 5 µg/ml of puromycin (SIGMA; P-8833) to establish an antigen expressing cell line (hROR1-full/293T, hROR1-EC/293T, hROR1-CK/293T, hROR1-CDR/293T) .

### (3) Preparation of Antigen for Immunization

Around 1 L of a supernatant obtained by culturing the above established expressing cell line (hROR1-EC/293T, hROR1-CK/293T, hROR1-CDR/293T) with DMEM-10% FBS (5µg/ml puromycin) or CD293 (Invitrogen) was collected, a myc-His tagged recombinant protein was purified from the collected supernatant by using an affinity column filled with carries chemically modified from a myc-tag antibody or a TALON Purification Kit (Clontech; K1253-1) and then dialyzed with PBS, and a purified protein was observed by using SDS-PAGE and western blot. A protein assay kit II (BioRad; 500-0002JA) was used to determine the protein concentration, and the antigen for immunization was prepared.

### (4) Immunization

A purified protein of a human ROR1 extracellular region (hROR1-EC), a purified protein of a partial length including the human ROR1 CDR-Kringle region (hROR1-CK), or a purified protein of a partial length including the human ROR1 CDR region (hROR1-CDR) were mixed with the same amount of complete adjuvant (SIGMA; F5881) to obtain an emulsion, and BALB/c mice (female) were immunized with the emulsion at 5 to 50 µg/head for several times every three to seven days. Further, in a case of immunization with the cell hROR1-full/293T in which the full length of a human ROR1 expressed, mice were immunized at 1 × 10⁵ cells/head for several times every three to seven days. Lymphocyte cells were extracted from the mice after three to five days from the final immunization, and cell fusion of the extracted lymphocyte cells with mouse myeloma cells P3U1 (P3-X63Ag8U1) was performed.

### (5) Cell Fusion and Selection and Acquisition of Monoclonal Antibody-producing Cell

Cell fusion was performed based on a general method illustrated below. For the fetal bovine serum (FBS) in all the culture media, a serum inactivated by a process of maintaining the temperature at 56 degrees Celsius for 30 minutes was used. The P3U1 was provided by being cultured with RPMI1640-10% FBS (containing penicillin, streptomycin). The extracted mouse lymphocyte cells and the P3U1 were mixed at a ratio of 10:1 to 2:1 and centrifuged. Then, 50% polyethylene glycol 4000 (Merck; 1.09727.0100) was gradually added as a fusion accelerating agent to the precipitated cells and gently mixed to perform cell fusion. Furthermore, after the RPMI1640 was gradually added and gently mixed, the mixture was centrifuged. The precipitated fused cells were diluted as appropriate with a HAT culture medium containing 15% FBS (containing RPMI1640, HAT-supplement (Invitrogen; 11067-030), penicillin, streptomycin) and seeded on a 96-well microplate at 200 µL/well.

The fused cells were cultured in a CO₂ incubator (5% CO₂, 37 degrees Celsius), and after a colony was sufficiently formed, a cultured supernatant was sampled and screened. In the screening, from those found to be positive in ELISA performed on a 96-well plate on which the ROR1 antigen used for immunization was sensitized, hybridomas were sorted with which response was observed by flow cytometry performed on the 293T in which the full length of the ROR1 was forcedly caused to express (hROR1-full/293T). After these hybridomas were amplified and cultured with an HT culture medium containing 15% FBS (containing RPMI1640, HT-supplement (Invitrogen; 21060-017), penicillin, streptomycin) and mono-cloned by a limiting dilution method. In such a way, 45 types of hybridoma clones (FIG. 1) were obtained which produce 19 clones from antibodies (a full-length protein of the extracellular region (amino acid positions 1 to 403) as an immunogenicity (FIG. 1: 2AM, 2AC, 2CC, 2AA, 2DC series), 13 clones from full-length expressing cells (hROR1-full/293T) as an immunogenicity (FIG. 1: 1AA, 1BA series), 5 clones from a partial-length protein including the CDR-Kringle region (amino acid positions 156 to 403) as an immunogenicity (FIG. 1: 6AM series), and 7 clones from a partial-length protein including the CDR region (amino acid positions 156 to 300) as an immunogenicity (FIG. 1: 70AF series). For these 45 types of antibodies, response to hROR1-EC, hROR1-CK, and hROR1-CDR was observed by the ELISA method, it was analyzed which region was recognized by each antibody to perform epitope grouping, and this results in that 23 types of antibodies recognize the Ig region, 17 types of antibodies recognize the CDR region, and 4 types of antibodies recognize the Kringle region (FIG. 1). Further, homology in the amino acid sequences of a mouse and a human is illustrated in FIG. 1. Although the homology differs for respective regions, the extracellular regions of the ROR1 of a mouse and a human exhibit a significantly high homology.

### (6) Response of Antibody to Human ROR1 Expressing Cell (FACS)

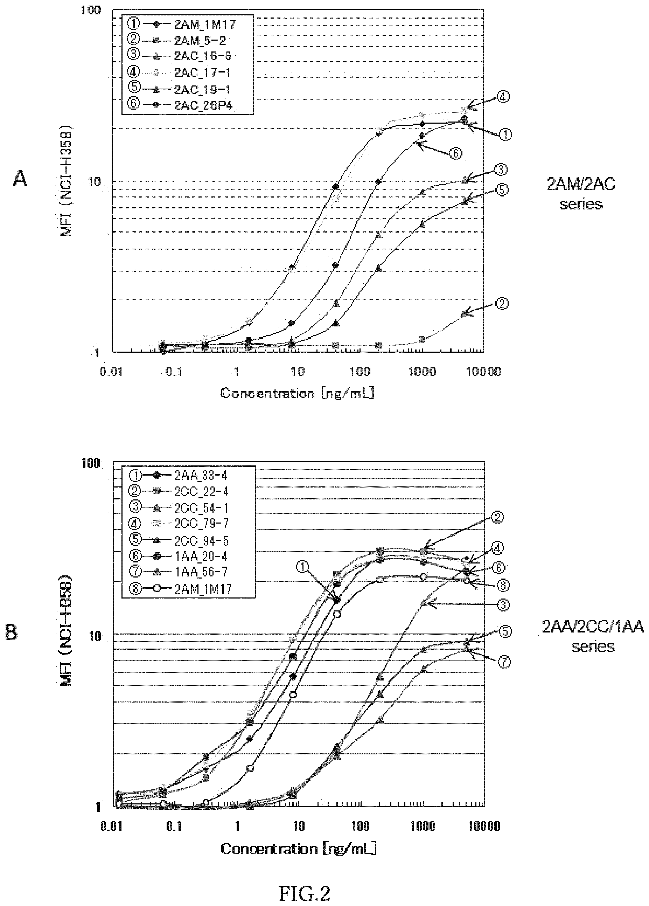

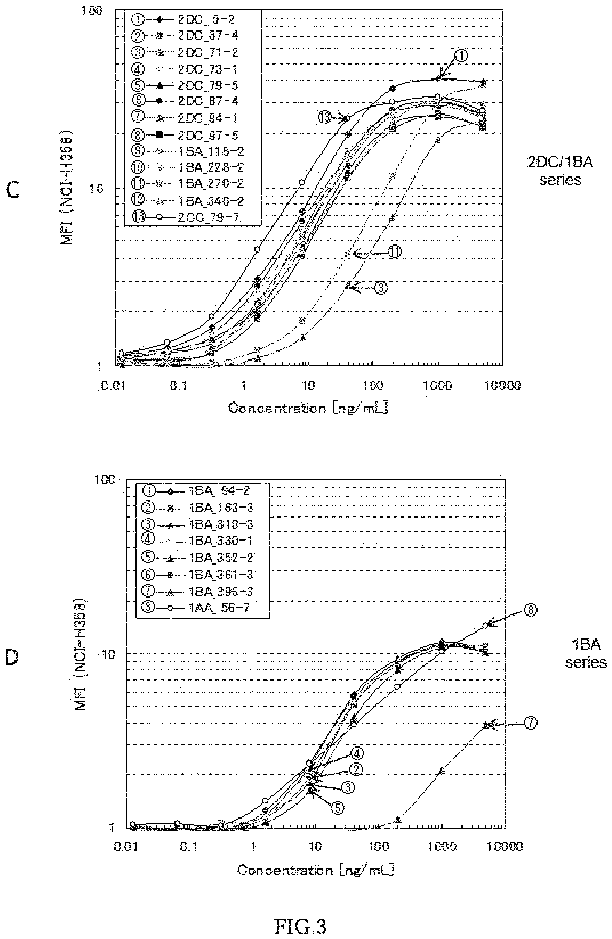

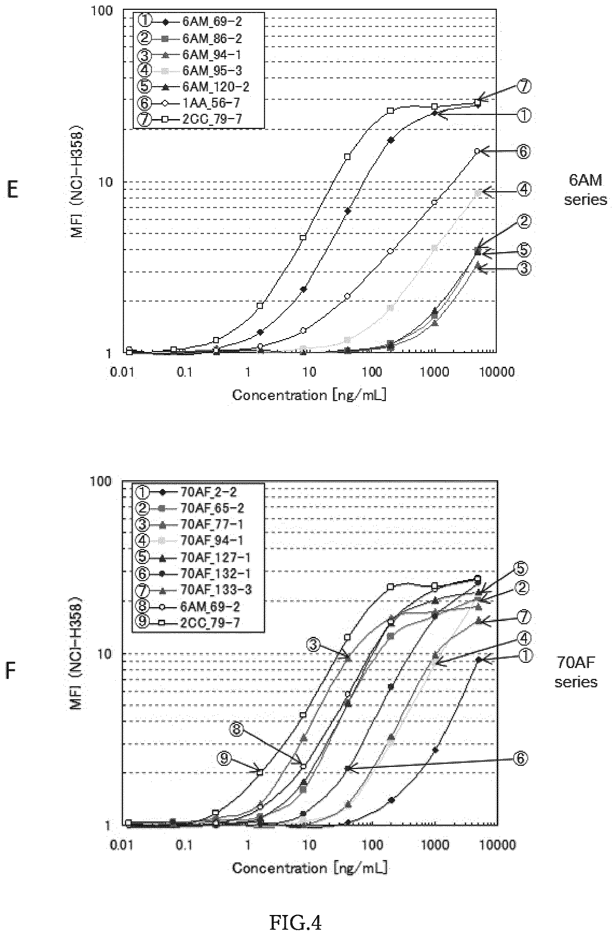

To inspect response of the antibodies obtained as described above to an endogenous human ROR1, response to an ROR1 high expressing cell NCI-H358 (ATCC accession No. CRL-5807) line was observed as below. The antibodies were purified from the culture supernatant of each hybridoma clone by a general affinity purification method using Protein A-Sepharose, respectively. The obtained purified antibodies were analyzed for the average fluorescence intensity of flow cytometry under the same condition (the same number of NCI-H358 (1 × 10⁵), the same concentration of respective purified antibodies, and the same concentration of secondary (detected) antibodies). Further, data was acquired also for the average fluorescence intensity that depends on the antibody concentration, the detection limit at a low concentration was analyzed, and thereby relative affinity was evaluated. FIG. 2 to FIG. 4 illustrate the results in a form of graphs. The horizontal axis of each graph represents the used antibody concentration (ng/mL), and the vertical axis represents the average fluorescence intensity (MFI) in flow cytometry. FIG. 2A is a diagram illustrating the response of anti-human ROR1 antibodies to ROR1 expressing cells of acquired monoclonal antibodies (2AM series and 2AC series). FIG. 2B is a diagram illustrating the response of anti-human ROR1 antibodies to ROR1 expressing cells of acquired monoclonal antibodies (2AA series, 2CC series, and 1AA series). FIG. 3C is a diagram illustrating the response of anti-human ROR1 antibodies to ROR1 expressing cells of acquired monoclonal antibodies (2DC series and some of 1BA series). FIG. 3D is a diagram illustrating the response of anti-human ROR1 antibodies to ROR1 expressing cells of acquired monoclonal antibodies (1BA series). FIG. 4E is a diagram illustrating the response of anti-human ROR1 antibodies to ROR1 expressing cells of acquired monoclonal antibodies (6AM series). FIG. 4F is a diagram illustrating the response of anti-human ROR1 antibodies to ROR1 expressing cells of acquired monoclonal antibodies (70AF series). Note that, in FIG. 3C and FIG. 3D, reference numbers of some of the acquired monoclonal antibodies are omitted because of the spacing between lines. As illustrated in FIG. 2 to FIG. 4, it was found that, although having different affinity, all the acquired antibodies are coupled to the human ROR1.

### (7) Isolation of VH Gene and VL Gene of Antibody and Identification of CDR

Next, two types of hybridomas were selected from hybridomas that produce antibodies which recognize the Ig region illustrated in FIG. 1, one type of hybridoma was selected from hybridomas that produce antibodies which recognize the CDR region illustrated in FIG. 1, and one type of hybridoma was selected from hybridomas that produce antibodies which recognize the Kringle region illustrated in FIG. 1. Note that the selected hybridomas are those having relatively high affinity to the ROR1 among antibodies that recognize respective regions. Next, the hybridomas that produce the selected antibodies were cultured, and the total RNA was acquired by a general method. Next, cDNA was acquired by 5'-RACE method using GeneRacer (registered trademark) kit (Invitrogen). The obtained cDNA was used as a template, and a PCR (35 cycles of a cycle [94 degrees Celsius for 30 seconds, 57 degrees Celsius for 30 seconds, and 72 degrees Celsius for 50 seconds]) was performed with Plutinum Tag High Fidelity (Invitrogen) using GeneRacer 5' Primer (SEQ ID No. 48: 5'-CGACTGGAGCACGAGGACACTGA-3') and CH1 (mouse IgG1 constant region 1) 3' Primer (SEQ ID No. 49: 5'-AATTTTCTTGTCCACCTGG-3'). Thereby, the VH gene (cDNA) was isolated. On the other hand, similarly for the VL gene, the PCR was performed by using GeneRacer 5' Primer and Ck (κ constant region) 3' Primer (SEQ ID No. 50: 5'-CTAACACTCATTCCTGTTGAAGCTCT-3'), and the VL gene (cDNA) was isolated.

After the VH gene and the VL gene were subcloned to a pT7Blue vector, respectively, an auto-sequencer (by Applied Biosystem) or a fluorescent auto-sequencer was used to analyze the sequence. Amino acids encoded by the resultant VH sequence and the resultant VL sequence and each CDR sequence were determined.

Respective identified sequences are indicated below. The details of the amino acid sequences are indicated in Table 1, and the details of the base sequences are indicated in Table 2 and Table 3.

### <2DC_5-2: recognize the Ig region>

### (amino acid sequences)

VH CDR1: SEQ ID No.: amino acid sequence indicated in No.8
VH CDR2: SEQ ID No.: amino acid sequence indicated in No.9
VH CDR3: SEQ ID No.: amino acid sequence indicated in No.10
VL CDR1: SEQ ID No.: amino acid sequence indicated in No.13
VL CDR2: SEQ ID No.: amino acid sequence indicated in No.14
VL CDR3: SEQ ID No.: amino acid sequence indicated in No.15
VH: SEQ ID No.: amino acid sequence indicated in No.7
VL: SEQ ID No.: amino acid sequence indicated in No.12

### (base sequence)

VH: SEQ ID No.: base sequence indicated in No.6
VL: SEQ ID No.: base sequence indicated in No.11

### <6AM_69-2: recognize the CDR region>

### (amino acid sequences)

VH CDR1: SEQ ID No.: amino acid sequence indicated in No.18
VH CDR2: SEQ ID No.: amino acid sequence indicated in No.19
VH CDR3: SEQ ID No.: amino acid sequence indicated in No.20
VL CDR1: SEQ ID No.: amino acid sequence indicated in No.23
VL CDR2: SEQ ID No.: amino acid sequence indicated in No.24
VL CDR3: SEQ ID No.: amino acid sequence indicated in No.25
VH: SEQ ID No.: amino acid sequence indicated in No.17
VL: SEQ ID No.: amino acid sequence indicated in No.22

### (base sequences)

VH: SEQ ID No.: base sequence indicated in No.16
VL: SEQ ID No.: base sequence indicated in No.21

### <6AM_120-2: recognize the Kringle region>

### (amino acid sequences)

VH CDR1: SEQ ID No.: amino acid sequence indicated in No.28
VH CDR2: SEQ ID No.: amino acid sequence indicated in No.29
VH CDR3: SEQ ID No.: amino acid sequence indicated in No.30
VL CDR1: SEQ ID No.: amino acid sequence indicated in No.33
VL CDR2: SEQ ID No.: amino acid sequence indicated in No.34
VL CDR3: SEQ ID No.: amino acid sequence indicated in No.35
VH: SEQ ID No.: amino acid sequence indicated in No.27
VL: SEQ ID No.: amino acid sequence indicated in No.32

### (base sequence)

VH: SEQ ID No.: base sequence indicated in No.26
VL: SEQ ID No.: base sequence indicated in No.31

### <2AM_1M17: recognize the Ig region>

### (amino acid sequences)

VH CDR1: SEQ ID No.: amino acid sequence indicated in No.38
VH CDR2: SEQ ID No.: amino acid sequence indicated in No.39
VH CDR3: SEQ ID No.: amino acid sequence indicated in No.40
VL CDR1: SEQ ID No.: amino acid sequence indicated in No.43
VL CDR2: SEQ ID No.: amino acid sequence indicated in No.44
VL CDR3: SEQ ID No.: amino acid sequence indicated in No.45
VH: SEQ ID No.: amino acid sequence indicated in No.37
VL: SEQ ID No.: amino acid sequence indicated in No.42

### (base sequences)

VH: SEQ ID No.: base sequence indicated in No.36
VL: SEQ ID No.: base sequence indicated in No.41

**[Table 1]**

| Clone name | SEQ ID No. | | amino acid sequences |
|---|---|---|---|
| 2DC_5-2 | 7 | VH | EVKLVESGGGLVKPGGSLKLSCAASGFTFSSYAMSWVRQTPEKRLEWVAAINTKGGSTYYPDTVKDRFTISR DNAKNILYLQMSSLRSEDTALYYCARHEPVYDYAMDYWGQGTSVTVSS |
| | 8 | VH CDR1 | SYAMS |
| | 9 | VH CDR2 | AINTKGGSTYYPDTVKD |
| | 10 | VH CDR3 | HEPVYDYAMDY |
| | 12 | VL | DIKMTQSPSSMYASLGERVTITCKASQDINSYLSWFQQKPGKSPKTLIYRANRLVDGVPSRFSGSGSGQDYSL TISSLECGDMGIYYCLQYDEFPYTFGGGTKLEIK |
| | 13 | VL CDR1 | KASQDINSYLS |
| | 14 | VL CDR2 | RANRLVD |
| | 15 | VL CDR3 | LQYDEFPYT |
| 6AM_69-2 | 17 | VH | QVQLQQSGAELARPGASVKLSCKASGYTFTSYGISWVKQRTGQGLEWIGEISPRSGYTYYNEKFKGKATLTA DKSSSTAYMQLSSLTSEDSAVYFCARPHYGDYVGYWGQGTTLTVSS |
| | 18 | VH CDR1 | SYGIS |
| | 19 | VH CDR2 | EISPRSGYTYYNEKFKG |
| | 20 | VH CDR3 | PHYGDYVGY |
| | 22 | VL | DVVMTQTPLSLPVSLGDQASISCRSSQSLVHSNGNTYLHWYLQKPGQSPKLLIYKVSNRFSGVPDRFSGSGS GTDFTLKISRVEAEDLGVYFCSQSTHVPFTFGTGTKLEIK |
| | 23 | VL CDR1 | RSSQSLVHSNGNTYLH |
| | 24 | VL CDR2 | KVSNRFS |
| | 25 | VL CDR3 | SQSTHVPFT |
| 6AM_120-2 | 27 | VH | EIQLQQSGAELVKPGASVKISCKASGYSFTGYNMNWVKQSHGKSLEWIGNINPYYGNTSYNQKFKGKATLTV DKSSSTAYMQLNSLTSEDSAVYYCAREGFEYFDYWGQGTTLTVSS |
| | 28 | VH CDR1 | GYNMN |
| | 29 | VH CDR2 | NINPYYGNTSYNQKFKG |
| | 30 | VH CDR3 | EGFEYFDY |
| | 32 | VL | DIQMTQSPASLSASVGETVTITCRASENIYSYLAWYQQKQGKSPQLLVYNVKTLAEGVPSRFSGSGSGTQFSL KINSLQPEDFGSYYCQHHYGIPYTFGSGTKLEIK |
| | 33 | VL CDR1 | RASENIYSYLA |
| | 34 | VL CDR2 | NVKTLAE |
| | 35 | VL CDR3 | QHHYGIPYT |
| 2AM_1M17 | 37 | VH | EVQLVESGGGLVKPGGSLKLSCAASGFTFSSYAMSWGRQTPEKRLEWVAAINSKGGSTYYPDTVKDRFTISR DNAKNTLYLQMSSLRSEDTALYYCARHDERIDYAMDYWGQGTSVTVSS |
| | 38 | VH CDR1 | SYAMS |
| | 39 | VH CDR2 | AINSKGGSTYYPDTVKD |
| | 40 | VH CDR3 | HDERIDYAMDY |
| | 42 | VL | DIKMTQSPSSMYASLGERVTITCKASQDINSYLSWFQQKPGKSPKTLIYRANRLVDGVPSRFSGSGSGQDYSL TISSLEYEDMGIYYCLQYDEFPYTFGSGTKLEIK |
| | 43 | VL CDR1 | KASQDINSYLS |
| | 44 | VL CDR2 | RANRLVD |
| | 45 | VL CDR3 | LQYDEFPYT |

**[Table 2]**

| Clone name | SEQ D No | | base sequences |
|---|---|---|---|
| 2DC_5-2 | 6 | VH | GAAGTGAAGCTGGTGGAGTCTGGGGGAGGCTTAGTGAAGCCTGGAGGGTCCCTGAAACTCTCCTGTGCAGCCTCTGGATTCACTTTCAGTAGCTATGCCATGT CTTGGGTTCGCCAGACTCCAGAGAAGAGGCTGGAGTGGGTCGCAGCCATTAATACTAAAGGTGGTAGCACCTACTATCCAGACACTGTGAAGGACCGATTCAC CATCTCCAGAGACAATGCCAAGAATATCCTGTATTTGCAAATGAGCAGTCTGAGGTCTGAGGACACAGCCTTGTATTACTGTGCAAGACACGAGCCGGTATACG ACTATGCTATGGACTACTGGGGTCAAGGAACCTCTGTCACCGTCTCCagcGCgTCCACCAAGGGCCCATCGGTCTTCCCCCTGGCACCCTCCTCCAAGAGCACC TCTGGGGGCACAGCGGCCCTGGGCTGCCTGGTCAAGGACTACTTCCCCGAACCGGTGACGGTGTCGTGGAACTCAGGCGCCCTGACCAGCGGCGTGCACAC CTTCCCGGCTGTCCTACAGTCCTCAGGACTCTACTCCCTCAGCAGCGTGGTGACCGTGCCCTCCAGCAGCTTGGGCACCCAGACCTACATCTGCAACGTGAAT CACAAGCCCAGCAACACCAAGGTGGACAAGAAAGTTGAGCCCAAATCTTGTGACAAAACTCACACATGCCCACCGTGCCCAGCACCTGAACTCCTGGGGGGAC CGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGA GGTCAAGTTCAACTGGTACGTGGACGGCGTGGAGGTGCATAATGCCAAGACAAAGCCGCGTGAGGAGCAGTACAACAGCACGTACCGTGTGGTCAGCGTCCT CACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAA GGGCAGCCCCGAGAACCACAGGTGTACACCCTGCCCCCATCCCGGGATGAGCTGACCAAGAACCAGGTCAGCCTGACCTGCCTGGTCAAAGGCTTCTATCCC AGCGACATCGCCGTGGAGTGGGAGAGCAATGGGCAGCCGGAGAACAACTACAAGACCACGCCTCCCGTGCTGGACTCCGACGGCTCCTTCTTCCTCTACAGC AAGCTCACCGTGGACAAGAGCAGGTGGCAGCAGGGGAACGTCTTCTCATGCTCCGTGATGCATGAGGCTCtGCACAACCACTACACGCAGAAGAGCCTCTCCC TGTCTCCGGGTAAAtag |
| | 11 | VL | GACATCAAGATGACCCAGTCTCCATCTTCCATGTATGCATCTCTAGGAGAGAGAGTCACTATCACTTGCAAGGCGAGTCAGGACATTAATAGCTATTTAAGCTGG TTCCAGCAGAAACCAGGGAAATCTCCTAAGACCCTGATCTATCGTGCAAACAGATTGGTAGATGGGGTCCCATCAAGGTTCAGTGGCAGTGGATCTGGGCAAGA TTATTCTCTCACCATCAGTAGCCTGGAGTGTGGAGATATGGGAATTTATTATTGTCTACAGTATGATGAGTTTCCGTACACGTTCGGAGGGGGGACCAAACTGGA AATAAAAcGAACTGTGGCTGCACCATCTGTCTTCATCTTCCCGCCATCTGATGAGCAGTTGAAATCTGGAACTGCCTCTGTTGTGTGCCTGCTGAATAACTTCTAT CCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGC CTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCT TCAACAGGGGAGAGTGTtga |
| 5AM_69-2 | 16 | VH | CAGGTTCAGCTGCAGCAGTCTGGAGCTGAATTGGCGAGGCCTGGGGCTTCAGTGAAGCTGTCCTGCAAGGCTTCTGGCTACACCTTCACAAGCTATGGTATAA GCTGGGTGAAGCAGAGAACTGGACAGGGCCTTGAGTGGATTGGAGAGATTTCTCCTAGAAGTGGTTATACCTACTACAATGAGAAGTTCAAGGGCAAGGCCAC ACTGACCGCAGACAAGTCCTCCAGCACAGCCTACATGCAGCTCAGCAGCCTGACATCTGAGGACTCTGCAGTCTATTTCTGTGCAAGACCTCACTATGGTGACT ACGTAGGCTACTGGGGCCAAGGCACCACTCTCACAGTCTCCagcGCgTCCACCAAGGGCCCATCGGTCTTCCCCCTGGCACCCTCCTCCAAGAGCACCTCTGGG GGCACAGCGGCCCTGGGCTGCCTGGTCAAGGACTACTTCCCCGAACCGGTGACGGTGTCGTGGAACTCAGGCGCCCTGACCAGCGGCGTGCACACCTTCCC GGCTGTCCTACAGTCCTCAGGACTCTACTCCCTCAGCAGCGTGGTGACCGTGCCCTCCAGCAGCTTGGGCACCCAGACCTACATCTGCAACGTGAATCACAAG CCCAGCAACACCAAGGTGGACAAGAAAGTTGAGCCCAAATCTTGTGACAAAACTCACACATGCCCACCGTGCCCAGCACCTGAACTCCTGGGGGGACCGTCAG TCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAA GTTCAACTGGTACGTGGACGGCGTGGAGGTGCATAATGCCAAGACAAAGCCGCGGGAGGAGCAGTACAACAGCACGTACCGTGTGGTCAGCGTCCTCACCGT CCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAG CCCCGAGAACCACAGGTGTACACCCTGCCCCCATCCCGGGATGAGCTGACCAAGAACCAGGTCAGCCTGACCTGCCTGGTCAAAGGCTTCTATCCCAGCGACA TCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCAC CGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCG GGTAAAtag |
| | 21 | VL | GATGTTGTGATGACCCAAACTCCACTCTCCCTGCCTGTCAGTCTTGGAGATCAAGCCTCCATCTCTTGCAGATCTAGTCAGAGCCTTGTACACAGTAATGGAAAC ACCTATTTACATTGGTACCTGCAGAAGCCAGGCCAGTCTCCAAAGCTCCTGATCTACAAAGTTTCCAACCGATTTTCTGGGGTCCCAGACAGGTTCAGTGGCAGT GGATCAGGGACAGATTTCACACTCAAGATCAGCAGAGTGGAGGCTGAGGATCTGGGAGTTTATTTCTGCTCTCAAAGTACACATGTTCCATTCACGTTCGGCAC GGGGACAAAATTGGAAATAAAAcGAACTGTGGCTGCACCATCTGTCTTCATCTTCCCGCCATCTGATGAGCAGTTGAAATCTGGAACTGCCTCTGTTGTGTGCCT GCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAG GACAGCACCTACAGCCTCAGCAGCACCCTGACGCTGAGCAAAGCAGACTACGAGAAACACAAAGTCTACGCCTGCGAAGTCACCCATCAGGGCCTGAGCTCGC CCGTCACAAAGAGCTTCAACAGGGGAGAGTGTtga |

**[Table 3]**

| Clone name | SEQ ID No. | | base sequences |
|---|---|---|---|
| 6AM_120-2 | 26 | VH | GAGATCCAGCTGCAGCAGTCTGGAGCTGAGTTGGTGAAGCCTGGGGCTTCAGTGAAGATATCCTGCAAGGCTTCTGGTTACTCATTCACTGGCTACAACATGA ACTGGGTGAAGCAGAGCCATGGAAAGAGCCTTGAGTGGATTGGAAATATTAATCCTTACTATGGTAATACTAGCTACAATCAGAAGTTCAAGGGCAAGGCCACA TTGACTGTAGACAAATCTTCCAGCACAGCCTACATGCAGCTCAACAGCCTGACATCTGAGGACTCTGCAGTCTATTACTGTGCAAGGGAGGGTTTTGAGTAtTTT GACTACTGGGGCCAAGGCACCACTCTCACAGTCTCCagcGCgTCCACCAAGGGCCCATCGGTCTTCCCCCTGGCACCCTCCTCCAAGAGCACCTCTGGGGGCA CAGCGGCCCTGGGCTGCCTGGTCAAGGACTACTTCCCCGAACCGGTGACGGTGTCGTGGAACTCAGGCGCCCTGACCAGCGGCGTGCACACCTTCCCGGCT GTCCTACAGTCCTCAGGACTCTACTCCCTCAGCAGCGTGGTGACCGTGCCCTCCAGCAGCTTGGGCACCCAGACCTACATCTGCAACGTGAATCACAAGCCCA GCAACACCAAGGTGGACAAGAAAGTTGAGCCCAAATCTTGTGACAAAACTCACACATGCCCACCGTGCCCAGCACCTGAACTCCTGGGGGGACCGTCAGTCTT CCTCTTCCCCCCAAAACCCAAGGACACCCTCATGATCTCCCGGACCCCTGAGGTCACATGCGTGGTGGTGGACGTGAGCCACGAAGACCCTGAGGTCAAGTT CAACTGGTACGTGGACGGCGTGGAGGTGCATAATGCCAAGACAAAGCCGCGTGAGGAGCAGTACAACAGCACGTACCGTGTGGTCAGCGTCCTCACCGTCCT GCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCC CCGAGAACCACAGGTGTACACCCTGCCCCCATCCCGGGATGAGCTGACCAAGAACCAGGTCAGCCTGACCTGCCTGGTCAAAGGCTTCTATCCCAGCGACAT CGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCAC CGTGGACAAGAGCAGGTGGCAGCAGGGGAACGTCTTCTCATGCTCCGTGATGCATGAGGCTCtGCACAACCACTACACGCAGAAGAGCCTGTCCCTGTCTCCG GGTAAAtag |
| | 31 | VL | GACATCCAGATGACTCAGTCTCCAGCCTCCCTATCTGCATCTGTGGGAGAAACTGTCACCATCACATGTCGAGCAAGTGAGAATATTTACAGTTATTTAGCATG GTATCAGCAGAAACAGGGAAAATCTCCTCAGCTCCTGGTCTATAATGTAAAAACCTTAGCAGAAGGTGTGCCATCAAGGTTCAGTGGCAGTGGATCAGGCACA CAGTTTTCTCTGAAGATCAACAGCCTGCAGCCTGAAGATTTTGGGAGTTATTACTGTCAACATCATTATGGTATTCCGTATACGTTCGGATCGGGGACCAAGCTG GAAATAAAAcGAACTGTGGCTGCACCATCTGTCTTCATCTTCCCGCCATCTGATGAGCAGTTGAAATCTGGAACTGCCTCTGTTGTGTGCCTGCTGAATAACTTC TATCCCAGAGAGGCCAAAGTACAGTGGAAGGTGGATAACGCCCTCCAATCGGGTAACTCCCAGGAGAGTGTCACAGAGCAGGACAGCAAGGACAGCACCTAC AGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAG AGCTTCAACAGGGGAGAGTGTtga |
| 2AM_1M17 | 36 | VH | GAGGTGCAGCTGGTGGAGTCTGGGGGAGGCTTAGTGAAGCCTGGAGGGTCCCTGAAACTCTCCTGTGCAGCCTCTGGATTCACTTTCAGTAGCTATGCCATG TCTTGGGTCCGCCAGACTCCAGAGAAGAGGCTGGAGTGGGTCGCAGCCATTAATAGTAAAGGTGGTAGCACCTACTATCCAGACACTGTGAAGGACCGATTCA CCATCTCCAGAGACAATGCCAAGAACACCCTGTACCTGCAAATGAGCAGTCTGAGGTCTGAGGACACAGCCTTGTATTACTGTGCAAGACATGATGAACGGATT GACTATGCTATGGACTACTGGGGTCAAGGAACCTCAGTCACCGTCTCCagcGCgTCCACCAAGGGCCCATCGGTCTTCCCCCTGGCACCCTCCTCCAAGAGCA CCTCTGGGGGCACAGCGGCCCTGGGCTGCCTGGTCAAGGACTACTTCCCCGAACCGGTGACGGTGTCGTGGAACTCAGGCGCCCTGACCAGCGGCGTGCAC ACCTTCCCGGCTGTCCTACAGTCCTCAGGACTCTACTCCCTCAGCAGCGTGGTGACCGTGCCCTCCAGCAGCTTGGGCACCCAGACCTACATCTGCAACGTGA ATCACAAGCCCAGCAACACCAAGGTGGACAAGAAAGTTGAGCCCAAATCTTGTGACAAAACTCACACATGCCCACCGTGCCCAGCACCTGAACTCCTGGGGGG ACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCT GAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCA AAGGGCAGCCCCGAGAACCACAGGTGTACACCCTGCCCCCATCCCGGGATGAGCTGACCAAGAACCAGGTCAGCCTGACCTGCCTGGTCAAAGGCTTCTATC CCAGCGACATCGCCGTGGAGTGGGAGAGCAATGGGCAGCCGGAGAACAACTACAAGACCACGCCTCCCGTGCTGGACTCCGACGGCTCCTTCTTCCTCTACA GCAAGCTCACCGTGGACAAGAGCAGGTGGCAGCAGGGGAACGTCTTCTCATGCTCCGTGATGCATGAGGCTCTGCACAACCACTACACGCAGAAGAGCCTCT CCCTGTCTCCGGGTAAAtag |
| | 41 | VL | GACATCAAGATGACCCAGTCTCCATCTTCCATGTATGCATCTCTAGGAGAGAGAGTCACTATCACTTGCAAGGCGAGTCAGGACATTAATAGCTATTTAAGCTG GTTCCAGCAGAAACCAGGGAAATCTCCTAAGACCCTGATCTATCGTGCAAACAGATTGGTAGATGGGGTCCCATCAAGGTTCAGTGGCAGTGGATCTGGGCAA GATTATTCTCTCACCATCAGCAGCCTGGAGTATGAAGATATGGGAATTTATTATTGTCTACAGTATGATGAGTTTCCGTATACGTTCGGATCGGGGACCAAGCTG GAAATAAAAcGAACTGTGGCTGCACCATCTGTCTTCATCTTCCCGCCATCTGATGAGCAGTTGAAATCTGGAACTGCCTCTGTTGTGTGCCTGCTGAATAACTTC TATCCCAGAGAGGCCAAAGTACAGTGGAAGGTGGATAACGCCCTCCAATCGGGTAACTCCCAGGAGAGTGTCACAGAGCAGGACAGCAAGGACAGCACCTAC AGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAG AGCTTCAACAGGGGAGAGTGTtga |

### (8) Creation of Chimerized Antibody

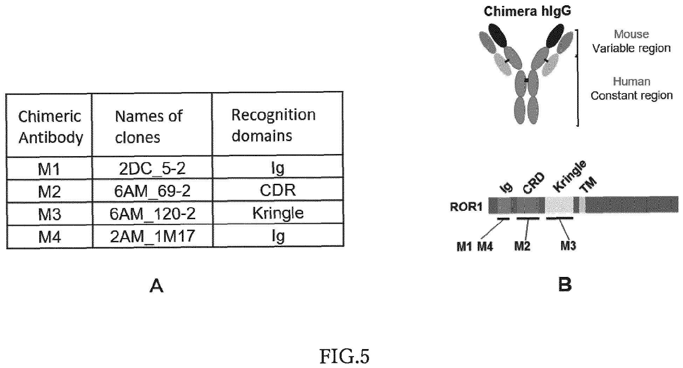

The VH region and the VL region were amplified by a PCR based on the identified gene sequence, and the PCR products were introduced in an antibody-producing vector in which the constant region of human IgG1 is incorporated by a conventional method. Then, 100 µl at 0.4 µg/µl of these expression vectors digested with a restriction enzyme ScaI and 1 × 10⁷ CHO cells (Chinese hamster ovary) were mixed in a cuvette, and transformed by an electroporation method. The mixture was suspended in a Ham F-12 culture medium containing 10% FBS (Thermo Fisher Scientific; 11765054) and dispensed on a 96-well microplate by 100 µl each, puromycin was added to have the final concentration of 20 µg/ml, and the resultant was cultured at 37 degrees Celsius for 12 days. The supernatants were fractionated from the wells forming a single colony, the clones having high activity with a sandwich ELISA method using an anti-human IgG antibody were sorted, the sorted clones were amplified and cultured into a 10 cm dish, the amplified and cultured clones were then acclimatized to an EX-CELL (registered trademark) CD-CHO Fusion culture medium (SAFC; 14365C) to which 4 mM of L-Alanyl-L-Glutamine (Wako; 016-21841) was added, and chimeric antibody-producing cell lines were established. The obtained cell lines were mass-cultured at 37 degrees Celsius with 5% CO₂ for 10 days by using the EX-CELL CD-CHO Fusion culture medium to which 4 mM of L-Alanyl-L-Glutamine was added, and purified antibodies of chimerized antibodies were obtained from the cultured supernatants by using Protein A Sepharose. As illustrated in FIG. 5A, hereafter, the chimerized antibodies obtained from clones 2DC_5-2, 6AM_69-2, 6AM_120-2, and 2AM_1M17 are denoted as M1, M2, M3, and M4, respectively. Further, FIG. 5B illustrates the structure and recognition domains of the created chimerized antibody.

### (9) Evaluation of Response of Chimeric Antibody to Human ROR1 Expressing Cell

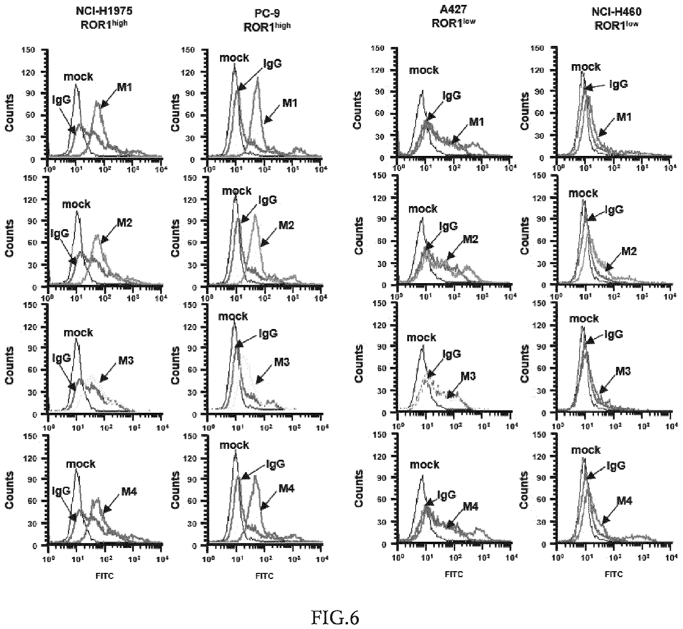

To inspect response of antibodies obtained as described above to an endogenous human ROR1, the response to the ROR1 high expressing cell line (NCI-H1975, PC-9) and the ROR1 low expressing cell line (A427, NCI-H460) was observed as follows. Note that NCI-H1975 (ATCC accession No. CRL-5908), A427 (ATCC accession No. HTB-53), and NCI-H460 (ATCC accession No. HTB-177) were purchased from American Type Culture Collection. The PC-9 cell line (RCB 4455) was obtained from RIKEN cell bank. Various cell lines were seeded on 6-well dishes to have 2 × 10⁵/well, cultured at 37 degrees Celsius for 24 hours, and collected after PBS washing. After chimerized antibodies labeled by using Alexa Fluor (registered trademark) 488 Antibody Labeling Kit (Thermo Fisher Scientific; A20181) were diluted to 1 µg/ml and reacted on ice for one hour, the resultant was washed with a PBS, and the reaction intensity was analyzed by flow cytometry. As a control, isotype control IgG1 against an antigen that does not exist in mammal cells was used. FIG. 6 illustrates graphs indicating the response of chimerized antibodies to the ROR1 high expressing cell line and the ROR1 low expressing cell line. As illustrated in FIG. 6, a reaction was observed in the chimeric antibodies M1, M2, M3, and M4 in the ROR1 high expressing cell line, and the reaction was significantly weak in the ROR1 low expressing cell line. From the above results, it was revealed that all the chimeric antibodies M1, M2, M3, and M4 exhibit response to the endogenous human ROR1.

### (10) Evaluation of Endocytosis of Chimeric Antibody into Cell

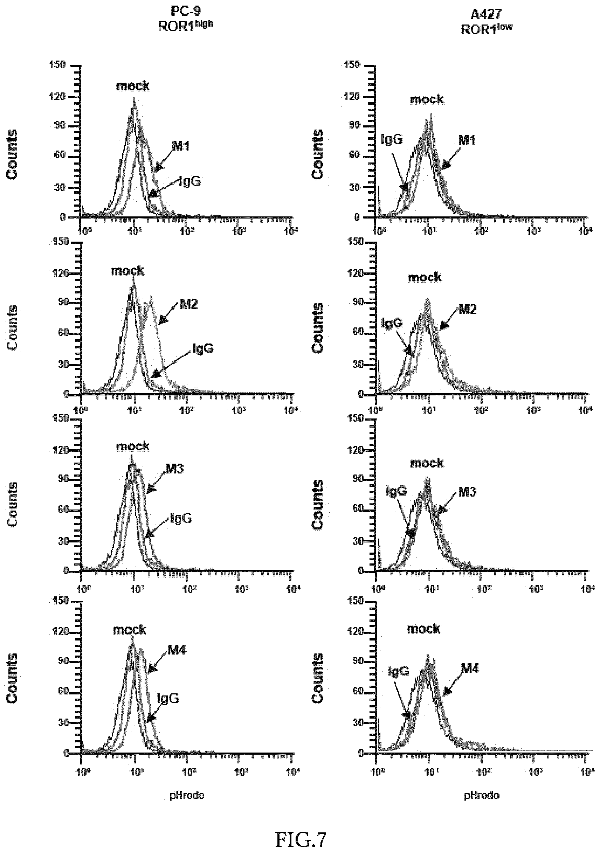

Next, to examine endocytosis activity of chimeric antibodies reacted with the human ROR1 into cells, the chimeric antibodies M1 to M4 were recognized by an acid pH-sensitive dye by using pHrodo (registered trademark) Red Microscale Labeling Kit (Thermo Fisher Scientific; P35363). For the ROR1 high expressing cell line PC-9 and the ROR1 low expressing cell line A427, cells at 1 × 10⁵/well were seeded on a 6-well dish having a cover glass therein, pHrodo labeled chimeric antibodies at 5 µg/ml were exposed, reacted at 37 degrees Celsius for 72 hours, and collected after PBS washing, and the fluorescence intensity was measured by flow cytometry. FIG. 7 is a diagram illustrating endocytosis of chimerized antibodies to the ROR1 high expressing cell and the ROR1 low expressing cell. As illustrated in FIG. 7, in the ROR1 high expressing cell line, endocytosis activity due to chimeric antibodies was observed, and when chimeric antibody M2 among others was used, particularly intense endocytosis was observed.

### (11) Evaluation of Cell Proliferation Suppression Effect Using Secondary Antibody Having Coupled Chimeric Antibody and Cytotoxic Drug

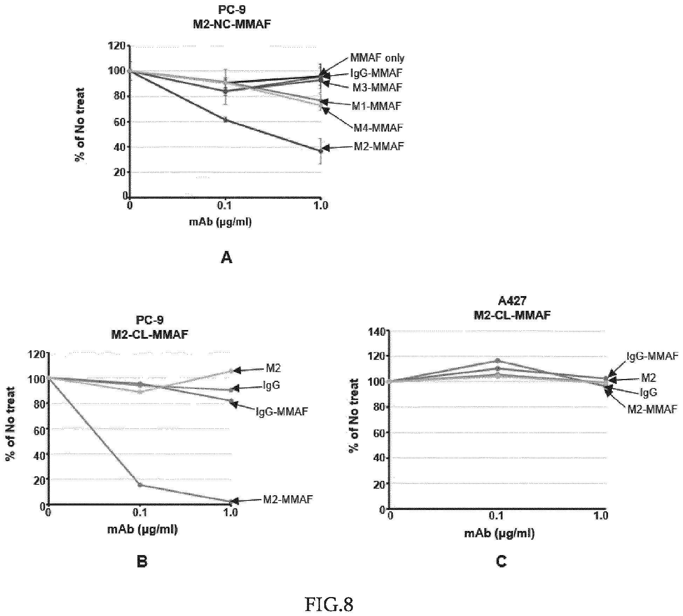

(a) To examine the effect of suppressing cell proliferation using endocytosis activity of chimeric antibodies into cells, a suppressing effect caused by a co-process with chimeric antibodies against cell proliferation was evaluated by using a secondary antibody in which monomethyl auristatin F (MMAF) that is a cytotoxic drug is coupled by a non-cleavage type linker. PC-9 lung adenocarcinoma cell lines were seeded on a 96-well dish at 1 × 10³ cells/well, a secondary antibody αHFc-NC-MMAF (Moradec; AH-101AF) in which 0.1 µg/ml or 1.0 µg/ml of the chimeric antibodies M1 to M4 and the MMAF were coupled by a non-cleavage type linker was added in the next day and cultured at 37 degrees Celsius. After five days from the start of the antibody addition process, influence on cell proliferation was studied by using the MTT assay method. FIG. 8A is a diagram illustrating cell proliferation inhibition of the PC-9 by the chimerized antibody and the non-cleavage type MMAF. As illustrated in FIG. 8A, it was revealed that, while the chimeric antibodies M1 and M4 that recognize the Ig region inhibit cell proliferation when the antibody concentration was high, the chimeric antibody M2 that recognizes the CDR region more intensively inhibits cell proliferation than M1 and M4. Further, it was revealed that all of M1, M2, M4 inhibits cell proliferation in a manner depending on the antibody concentration. On the other hand, the chimeric antibody M3 that recognizes the Kringle region did not at all inhibit cell proliferation.
(b) Next, to further examine the cell proliferation suppression effect of the chimeric antibody M2 that inhibited cell proliferation the most intensively in (a) described above, αHFc-CL-MMAF (Moradec; AH-102AF) in which the cytotoxic drug MMAF is coupled to the secondary antibody by a cleavage type linker was used to perform a co-process of the chimeric antibody M2 and the MMAF-coupled secondary antibody, and influence on the cell proliferation was evaluated. The ROR1 high expressing cell line, namely, the PC-9 cell line and the ROR1 low expressing cell line, namely, the A427 cell line were seeded on a 96-well dish at 1 × 10³ cells/well, and 0.1 µg/ml or 1.0 µg/ml of the chimeric antibody M2 and the αHFc-CL-MMAF were added together in the next day, and cultured at 37 degrees Celsius for five days, and suppression activity against cell proliferation was then measured by an MTT assay method. FIG. 8B is a diagram illustrating the cell proliferation inhibition of the PC-9 by the chimerized antibody M2 and the cleavage type MMAF. FIG. 8C is a diagram illustrating the cell proliferation inhibition of the A427 by the chimerized antibody M2 and the cleavage type MMAF.

As illustrated in FIG. 8B, in the PC-9 cell line, while cell proliferation was significantly suppressed when the chimeric antibody M2 and the MMAF-coupled secondary antibody were co-processed, no suppression of cell proliferation was found in the control (only IgG, only M2, IgG-MMAF). Further, it was revealed that the cleavage type linker can more suppress cell proliferation than the non-cleavage type linker. On the other hand, as illustrated in FIG. 8C, in the A427 that is the ROR1 low expressing cell line, even when the chimeric antibody M2 and the MMAF-coupled secondary antibody were co-processed, suppression of cell proliferation was not found at all.

From the above results, it was revealed that:
(1) Any antibodies that recognize the extracellular region of the human ROR1 are specifically coupled to the human ROR1;
(2) However, antibodies that recognize positions 156 to 300 in the amino acid sequence as an epitope exhibit more significant endocytosis into cancer cells than antibodies that recognize other regions as an epitope; and
(3) Therefore, antibodies that recognize positions 156 to 300 in the amino acid sequence as an epitope can be used as an antibody for delivering a drug into a cancer cell and a pharmaceutical composition when coupled with a drug.

### [Industrial Applicability]

An antibody that recognizes positions 156 to 300 in the amino acid sequence as an epitope is endocytosed in a cell and thus can be used as a drug delivery antibody or a pharmaceutical composition. Therefore, such an antibody is useful in pharmaceutical industries.

## Claims

1. An anti-ROR1 monoclonal antibody that recognizes positions 156 to 300 in the amino acid sequence of a human ROR1 protein (GenBank accession No. NP_005003.2) as an epitope.

2. The anti-ROR1 monoclonal antibody according to claim 1, wherein the anti-ROR1 monoclonal antibody is specifically coupled to an ROR1 of a human cancer cell and endocytosed in a cell by endocytosis.

3. The anti-ROR1 monoclonal antibody according to claim 1 or 2,
wherein a heavy-chain variable region includes a region consisting of
a CDR1 H region consisting of an amino acid sequence of SYGIS (SEQ ID No. 18),
a CDR2 H region consisting of an amino acid sequence of EISPRSGYTYYNEKFKG (SEQ ID No. 19), and
a CDR3 H region consisting of an amino acid sequence of PHYGDYVGY (SEQ ID No. 20), and
wherein a light-chain variable region includes a region consisting of
a CDR1 L region consisting of an amino acid sequence of RSSQSLVHSNGNTYLH (SEQ ID No. 23),
a CDR2 L region consisting of an amino acid sequence of KVSNRFS (SEQ ID No. 24), and
a CDR3 L region consisting of an amino acid sequence of SQSTHVPFT (SEQ ID No. 25).

4. The anti-ROR1 monoclonal antibody according to any one of claims 1 to 3,
wherein the amino acid sequence of an H-chain variable region is of SEQ ID No. 17, and
wherein the amino acid sequence of an L-chain variable region is of SEQ ID No. 22.

5. A functional fragment of the anti-ROR1 monoclonal antibody according to any one of claims 1 to 4.

6. A gene having, as an open reading frame region, a gene that encodes the CDR sequence according to claim 3 or the variable region according to claim 4.

7. A drug delivery composition including the anti-ROR1 monoclonal antibody according to any one of claims 1 to 4 or the functional fragment of the anti-ROR1 monoclonal antibody according to claim 5.

8. A pharmaceutical composition including:
the anti-ROR1 monoclonal antibody according to any one of claims 1 to 4 or the functional fragment of the anti-ROR1 monoclonal antibody according to claim 5;
a linker; and
a drug,
wherein one terminal of the linker is coupled to the anti-ROR1 monoclonal antibody or the functional fragment, and the other terminal of the linker is coupled to the drug.


SEQUENCE LISTING

<110>  National University Corporation Nagoya University

<120>  Anti-ROR1 monoclonal antibody and functional fragment thereof,
       gene, drug delivery composition, and pharmaceutical composition

<130>  M17093PCT

<160>  50

<170>  PatentIn version 3.5

<210>  1
<211>  31
<212>  DNA
<213>  Artificial Sequence

<220>
<223>  Artificially synthesized primer sequence

<400>  1
aatagcggcc gcaccatgca ccggccgcgc c                                    31


<210>  2
<211>  35
<212>  DNA
<213>  Artificial Sequence

<220>
<223>  Artificially synthesized primer sequence

<400>  2
gcggctcgag cagttctgca gaaatcatag attcg                                35


<210>  3
<211>  33
<212>  DNA
<213>  Artificial Sequence

<220>
<223>  Artificially synthesized primer sequence

<400>  3
ccgcggatcc ttccatttta ttcttctcct tgg                                  33


<210>  4
<211>  33
<212>  DNA
<213>  Artificial Sequence

<220>
<223>  Artificially synthesized primer sequence

<400>  4
aatagcggcc gcaagtccag gatactcaga tga                                  33


<210>  5
<211>  29

<212>  DNA
<213>  Artificial Sequence

<220>
<223>  artificially synthesized primer sequence

<400>  5
agcaggatcc aatccggata cagttcgca                                        29


<210>  6
<211>  1353
<212>  DNA
<213>  Mus musculus

<400>  6
gaagtgaagc tggtggagtc tgggggaggc ttagtgaagc ctggagggtc cctgaaactc      60

tcctgtgcag cctctggatt cactttcagt agctatgcca tgtcttgggt tcgccagact     120

ccagagaaga ggctggagtg ggtcgcagcc attaatacta aaggtggtag cacctactat     180

ccagacactg tgaaggaccg attcaccatc tccagagaca atgccaagaa tatcctgtat     240

ttgcaaatga gcagtctgag gtctgaggac acagccttgt attactgtgc aagacacgag     300

ccggtatacg actatgctat ggactactgg ggtcaaggaa cctctgtcac cgtctccagc     360

gcgtccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgtgagga gcagtacaac     900

agcacgtacc gtgtggtcag cgtcctcacc gtcctgcacc aggactggct gaatggcaag     960

gagtacaagt gcaaggtctc caacaaagcc ctcccagccc ccatcgagaa aaccatctcc    1020

aaagccaaag ggcagccccg agaaccacag gtgtacaccc tgcccccatc ccgggatgag    1080

ctgaccaaga accaggtcag cctgacctgc ctggtcaaag gcttctatcc cagcgacatc    1140

gccgtggagt gggagagcaa tgggcagccg gagaacaact acaagaccac gcctcccgtg    1200

ctggactccg acggctcctt cttcctctac agcaagctca ccgtggacaa gagcaggtgg    1260

cagcagggga acgtcttctc atgctccgtg atgcatgagg ctctgcacaa ccactacacg    1320

cagaagagcc tctccctgtc tccgggtaaa tag                                 1353

<210>  7
<211>  120
<212>  PRT
<213>  Mus musculus

<400>  7

Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Arg Leu Glu Trp Val
        35                  40                  45

Ala Ala Ile Asn Thr Lys Gly Gly Ser Thr Tyr Tyr Pro Asp Thr Val
    50                  55                  60

Lys Asp Arg Phe Thr Ile Ser Arg Asp Asn Ala Lys Asn Ile Leu Tyr
65                  70                  75                  80

Leu Gln Met Ser Ser Leu Arg Ser Glu Asp Thr Ala Leu Tyr Tyr Cys
                85                  90                  95

Ala Arg His Glu Pro Val Tyr Asp Tyr Ala Met Asp Tyr Trp Gly Gln
            100                 105                 110

Gly Thr Ser Val Thr Val Ser Ser
        115                 120


<210>  8
<211>  5
<212>  PRT
<213>  Mus musculus

<400>  8

Ser Tyr Ala Met Ser
1               5


<210>  9
<211>  17
<212>  PRT
<213>  Mus musculus

<400>  9

Ala Ile Asn Thr Lys Gly Gly Ser Thr Tyr Tyr Pro Asp Thr Val Lys
1               5                   10                  15

Asp

<210>  10
<211>  11
<212>  PRT
<213>  Mus musculus

<400>  10

His Glu Pro Val Tyr Asp Tyr Ala Met Asp Tyr
1               5                   10


<210>  11
<211>  645
<212>  DNA
<213>  Mus musculus

<400>  11
gacatcaaga tgacccagtc tccatcttcc atgtatgcat ctctaggaga gagagtcact        60

atcacttgca aggcgagtca ggacattaat agctatttaa gctggttcca gcagaaacca       120

gggaaatctc ctaagaccct gatctatcgt gcaaacagat tggtagatgg ggtcccatca       180

aggttcagtg gcagtggatc tgggcaagat tattctctca ccatcagtag cctggagtgt       240

ggagatatgg gaatttatta ttgtctacag tatgatgagt ttccgtacac gttcggaggg       300

gggaccaaac tggaaataaa acgaactgtg gctgcaccat ctgtcttcat cttcccgcca       360

tctgatgagc agttgaaatc tggaactgcc tctgttgtgt gcctgctgaa taacttctat       420

cccagagagg ccaaagtaca gtggaaggtg gataacgccc tccaatcggg taactcccag       480

gagagtgtca cagagcagga cagcaaggac agcacctaca gcctcagcag caccctgacg       540

ctgagcaaag cagactacga gaaacacaaa gtctacgcct gcgaagtcac ccatcagggc       600

ctgagctcgc ccgtcacaaa gagcttcaac aggggagagt gttga                       645


<210>  12
<211>  107
<212>  PRT
<213>  Mus musculus

<400>  12

Asp Ile Lys Met Thr Gln Ser Pro Ser Ser Met Tyr Ala Ser Leu Gly
1               5                   10                  15


Glu Arg Val Thr Ile Thr Cys Lys Ala Ser Gln Asp Ile Asn Ser Tyr
            20                  25                  30


Leu Ser Trp Phe Gln Gln Lys Pro Gly Lys Ser Pro Lys Thr Leu Ile
        35                  40                  45


Tyr Arg Ala Asn Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Cys
65                  70                  75                  80

Gly Asp Met Gly Ile Tyr Tyr Cys Leu Gln Tyr Asp Glu Phe Pro Tyr
                85                  90                  95

Thr Phe Gly Gly Gly Thr Lys Leu Glu Ile Lys
            100                 105

<210>  13
<211>  11
<212>  PRT
<213>  Mus musculus

<400>  13

Lys Ala Ser Gln Asp Ile Asn Ser Tyr Leu Ser
1               5                   10

<210>  14
<211>  7
<212>  PRT
<213>  Mus musculus

<400>  14

Arg Ala Asn Arg Leu Val Asp
1               5

<210>  15
<211>  9
<212>  PRT
<213>  Mus musculus

<400>  15

Leu Gln Tyr Asp Glu Phe Pro Tyr Thr
1               5

<210>  16
<211>  1347
<212>  DNA
<213>  Mus musculus

<400>  16
caggttcagc tgcagcagtc tggagctgaa ttggcgaggc ctggggcttc agtgaagctg      60

tcctgcaagg cttctggcta caccttcaca agctatggta taagctgggt gaagcagaga     120

actggacagg gccttgagtg gattggagag atttctccta gaagtggtta tacctactac     180

aatgagaagt tcaagggcaa ggccacactg accgcagaca agtcctccag cacagcctac     240

atgcagctca gcagcctgac atctgaggac tctgcagtct atttctgtgc aagacctcac     300

tatggtgact acgtaggcta ctggggccaa ggcaccactc tcacagtctc cagcgcgtcc 360

accaagggcc catcggtctt ccccctggca ccctcctcca agagcacctc tgggggcaca 420

gcggccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcaccca gacctacatc 600

tgcaacgtga atcacaagcc cagcaacacc aaggtggaca agaaagttga gcccaaatct 660

tgtgacaaaa ctcacacatg cccaccgtgc ccagcacctg aactcctggg gggaccgtca 720

gtcttcctct tccccccaaa acccaaggac accctcatga tctcccggac ccctgaggtc 780

acatgcgtgg tggtggacgt gagccacgaa gaccctgagg tcaagttcaa ctggtacgtg 840

gacggcgtgg aggtgcataa tgccaagaca aagccgcgtg aggagcagta caacagcacg 900

taccgtgtgg tcagcgtcct caccgtcctg caccaggact ggctgaatgg caaggagtac 960

aagtgcaagg tctccaacaa agccctccca gcccccatcg agaaaaccat ctccaaagcc 1020

aaagggcagc cccgagaacc acaggtgtac accctgcccc catcccggga tgagctgacc 1080

aagaaccagg tcagcctgac ctgcctggtc aaaggcttct atcccagcga catcgccgtg 1140

gagtgggaga gcaatgggca gccggagaac aactacaaga ccacgcctcc cgtgctggac 1200

tccgacggct ccttcttcct ctacagcaag ctcaccgtgg acaagagcag gtggcagcag 1260

gggaacgtct tctcatgctc cgtgatgcat gaggctctgc acaaccacta cacgcagaag 1320

agcctctccc tgtctccggg taaatag 1347

<210> 17
<211> 118
<212> PRT
<213> Mus musculus

<400> 17

Gln Val Gln Leu Gln Gln Ser Gly Ala Glu Leu Ala Arg Pro Gly Ala
1               5                   10                  15

Ser Val Lys Leu Ser Cys Lys Ala Ser Gly Tyr Thr Phe Thr Ser Tyr
            20                  25                  30

Gly Ile Ser Trp Val Lys Gln Arg Thr Gly Gln Gly Leu Glu Trp Ile
        35                  40                  45

Gly Glu Ile Ser Pro Arg Ser Gly Tyr Thr Tyr Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Pro His Tyr Gly Asp Tyr Val Gly Tyr Trp Gly Gln Gly Thr
            100                 105                 110

Thr Leu Thr Val Ser Ser
        115

<210>  18
<211>  5
<212>  PRT
<213>  Mus musculus

<400>  18

Ser Tyr Gly Ile Ser
1               5

<210>  19
<211>  17
<212>  PRT
<213>  Mus musculus

<400>  19

Glu Ile Ser Pro Arg Ser Gly Tyr Thr Tyr Tyr Asn Glu Lys Phe Lys
1               5                   10                  15

Gly

<210>  20
<211>  9
<212>  PRT
<213>  Mus musculus

<400>  20

Pro His Tyr Gly Asp Tyr Val Gly Tyr
1               5

<210>  21
<211>  660
<212>  DNA
<213>  Mus musculus

<400>  21
gatgttgtga tgacccaaac tccactctcc ctgcctgtca gtcttggaga tcaagcctcc      60

atctcttgca gatctagtca gagccttgta cacagtaatg gaaacaccta tttacattgg     120

tacctgcaga agccaggcca gtctccaaag ctcctgatct acaaagtttc caaccgattt     180

tctggggtcc cagacaggtt cagtggcagt ggatcaggga cagatttcac actcaagatc     240

agcagagtgg aggctgagga tctgggagtt tatttctgct ctcaaagtac acatgttcca      300

ttcacgttcg gcacggggac aaaattggaa ataaaacgaa ctgtggctgc accatctgtc      360

ttcatcttcc cgccatctga tgagcagttg aaatctggaa ctgcctctgt tgtgtgcctg      420

ctgaataact tctatcccag agaggccaaa gtacagtgga aggtggataa cgccctccaa      480

tcgggtaact cccaggagag tgtcacagag caggacagca aggacagcac ctacagcctc      540

agcagcaccc tgacgctgag caaagcagac tacgagaaac acaaagtcta cgcctgcgaa      600

gtcacccatc agggcctgag ctcgcccgtc acaaagagct tcaacagggg agagtgttga      660

<210>  22
<211>  112
<212>  PRT
<213>  Mus musculus

<400>  22

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Phe Thr Phe Gly Thr Gly Thr Lys Leu Glu Ile Lys
            100                 105                 110

<210>  23
<211>  16
<212>  PRT
<213>  Mus musculus

<400>  23

Arg Ser Ser Gln Ser Leu Val His Ser Asn Gly Asn Thr Tyr Leu His
1               5                   10                  15

<210>  24

<211>  7
<212>  PRT
<213>  Mus musculus

<400>  24

Lys Val Ser Asn Arg Phe Ser
1               5


<210>  25
<211>  9
<212>  PRT
<213>  Mus musculus

<400>  25

Ser Gln Ser Thr His Val Pro Phe Thr
1               5


<210>  26
<211>  1344
<212>  DNA
<213>  Mus musculus

<400>  26
gagatccagc tgcagcagtc tggagctgag ttggtgaagc ctggggcttc agtgaagata      60

tcctgcaagg cttctggtta ctcattcact ggctacaaca tgaactgggt gaagcagagc     120

catggaaaga gccttgagtg gattggaaat attaatcctt actatggtaa tactagctac     180

aatcagaagt tcaagggcaa ggccacattg actgtagaca aatcttccag cacagcctac     240

atgcagctca acagcctgac atctgaggac tctgcagtct attactgtgc aagggagggt     300

tttgagtatt ttgactactg gggccaaggc accactctca cagtctccag cgcgtccacc     360

aagggcccat cggtcttccc cctggcaccc tcctccaaga gcacctctgg gggcacagcg     420

gccctgggct gcctggtcaa ggactacttc cccgaaccgg tgacggtgtc gtggaactca     480

ggcgccctga ccagcggcgt gcacaccttc ccggctgtcc tacagtcctc aggactctac     540

tccctcagca gcgtggtgac cgtgccctcc agcagcttgg gcacccagac ctacatctgc     600

aacgtgaatc acaagcccag caacaccaag gtggacaaga aagttgagcc caaatcttgt     660

gacaaaactc acacatgccc accgtgccca gcacctgaac tcctgggggg accgtcagtc     720

ttcctcttcc ccccaaaacc caaggacacc ctcatgatct cccggacccc tgaggtcaca     780

tgcgtggtgg tggacgtgag ccacgaagac cctgaggtca agttcaactg gtacgtggac     840

ggcgtggagg tgcataatgc caagacaaag ccgcgtgagg agcagtacaa cagcacgtac     900

cgtgtggtca gcgtcctcac cgtcctgcac caggactggc tgaatggcaa ggagtacaag     960

tgcaaggtct ccaacaaagc cctcccagcc cccatcgaga aaaccatctc caaagccaaa    1020

gggcagcccc gagaaccaca ggtgtacacc ctgcccccat cccgggatga gctgaccaag    1080

aaccaggtca gcctgacctg cctggtcaaa ggcttctatc ccagcgacat cgccgtggag     1140

tgggagagca atgggcagcc ggagaacaac tacaagacca cgcctcccgt gctggactcc     1200

gacggctcct tcttcctcta cagcaagctc accgtggaca agagcaggtg gcagcagggg     1260

aacgtcttct catgctccgt gatgcatgag gctctgcaca accactacac gcagaagagc     1320

ctctccctgt ctccgggtaa atag                                            1344

<210>  27
<211>  117
<212>  PRT
<213>  Mus musculus

<400>  27

Glu Ile Gln Leu Gln Gln Ser Gly Ala Glu Leu Val Lys Pro Gly Ala
1               5                   10                  15

Ser Val Lys Ile Ser Cys Lys Ala Ser Gly Tyr Ser Phe Thr Gly Tyr
            20                  25                  30

Asn Met Asn Trp Val Lys Gln Ser His Gly Lys Ser Leu Glu Trp Ile
        35                  40                  45

Gly Asn Ile Asn Pro Tyr Tyr Gly Asn Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Asn Ser Leu Thr Ser Glu Asp Ser Ala Val Tyr Tyr Cys
                85                  90                  95

Ala Arg Glu Gly Phe Glu Tyr Phe Asp Tyr Trp Gly Gln Gly Thr Thr
            100                 105                 110

Leu Thr Val Ser Ser
        115

<210>  28
<211>  5
<212>  PRT
<213>  Mus musculus

<400>  28

Gly Tyr Asn Met Asn
1               5

<210>  29
<211>  17

<212>  PRT
<213>  Mus musculus

<400>  29

Asn Ile Asn Pro Tyr Tyr Gly Asn Thr Ser Tyr Asn Gln Lys Phe Lys
1               5                   10                  15

Gly

<210>  30
<211>  8
<212>  PRT
<213>  Mus musculus

<400>  30

Glu Gly Phe Glu Tyr Phe Asp Tyr
1               5

<210>  31
<211>  645
<212>  DNA
<213>  Mus musculus

<400>  31
gacatccaga tgactcagtc tccagcctcc ctatctgcat ctgtgggaga aactgtcacc      60

atcacatgtc gagcaagtga gaatatttac agttatttag catggtatca gcagaaacag     120

ggaaaatctc ctcagctcct ggtctataat gtaaaaacct tagcagaagg tgtgccatca     180

aggttcagtg gcagtggatc aggcacacag ttttctctga agatcaacag cctgcagcct     240

gaagattttg ggagttatta ctgtcaacat cattatggta ttccgtatac gttcggatcg     300

gggaccaagc tggaaataaa acgaactgtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gttga                     645

<210>  32
<211>  107
<212>  PRT
<213>  Mus musculus

<400>  32

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

Tyr Asn Val Lys Thr Leu Ala Glu Gly Val Pro Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Ser Tyr Tyr Cys Gln His His Tyr Gly Ile Pro Tyr
                85                  90                  95

Thr Phe Gly Ser Gly Thr Lys Leu Glu Ile Lys
            100                 105

<210>  33
<211>  11
<212>  PRT
<213>  Mus musculus

<400>  33

Arg Ala Ser Glu Asn Ile Tyr Ser Tyr Leu Ala
1               5                   10

<210>  34
<211>  7
<212>  PRT
<213>  Mus musculus

<400>  34

Asn Val Lys Thr Leu Ala Glu
1               5

<210>  35
<211>  9
<212>  PRT
<213>  Mus musculus

<400>  35

Gln His His Tyr Gly Ile Pro Tyr Thr
1               5

<210>  36
<211>  1353
<212>  DNA
<213>  Mus musculus

<400>  36
gaggtgcagc tggtggagtc tgggggaggc ttagtgaagc ctggagggtc cctgaaactc      60

tcctgtgcag cctctggatt cactttcagt agctatgcca tgtcttgggg tcgccagact     120

ccagagaaga ggctggagtg ggtcgcagcc attaatagta aaggtggtag cacctactat     180

ccagacactg tgaaggaccg attcaccatc tccagagaca atgccaagaa caccctgtac     240

ctgcaaatga gcagtctgag gtctgaggac acagccttgt attactgtgc aagacatgat     300

gaacggattg actatgctat ggactactgg ggtcaaggaa cctcagtcac cgtctccagc     360

gcgtccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgtgagga gcagtacaac     900

agcacgtacc gtgtggtcag cgtcctcacc gtcctgcacc aggactggct gaatggcaag     960

gagtacaagt gcaaggtctc caacaaagcc ctcccagccc ccatcgagaa aaccatctcc    1020

aaagccaaag ggcagccccg agaaccacag gtgtacaccc tgcccccatc ccgggatgag    1080

ctgaccaaga accaggtcag cctgacctgc ctggtcaaag gcttctatcc cagcgacatc    1140

gccgtggagt gggagagcaa tgggcagccg gagaacaact acaagaccac gcctcccgtg    1200

ctggactccg acggctcctt cttcctctac agcaagctca ccgtggacaa gagcaggtgg    1260

cagcagggga acgtcttctc atgctccgtg atgcatgagg ctctgcacaa ccactacacg    1320

cagaagagcc tctccctgtc tccgggtaaa tag                                 1353

<210>  37
<211>  120
<212>  PRT
<213>  Mus musculus

<400>  37

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Gly Arg Gln Thr Pro Glu Lys Arg Leu Glu Trp Val
        35                  40                  45

Ala Ala Ile Asn Ser Lys Gly Gly Ser Thr Tyr Tyr Pro Asp Thr Val
    50                  55                  60

Lys Asp Arg Phe Thr Ile Ser Arg Asp Asn Ala Lys Asn Thr Leu Tyr
65                  70                  75                  80

Leu Gln Met Ser Ser Leu Arg Ser Glu Asp Thr Ala Leu Tyr Tyr Cys
                85                  90                  95

Ala Arg His Asp Glu Arg Ile Asp Tyr Ala Met Asp Tyr Trp Gly Gln
            100                 105                 110

Gly Thr Ser Val Thr Val Ser Ser
        115                 120

<210>  38
<211>  5
<212>  PRT
<213>  Mus musculus

<400>  38

Ser Tyr Ala Met Ser
1               5

<210>  39
<211>  17
<212>  PRT
<213>  Mus musculus

<400>  39

Ala Ile Asn Ser Lys Gly Gly Ser Thr Tyr Tyr Pro Asp Thr Val Lys
1               5                   10                  15

Asp

<210>  40
<211>  11
<212>  PRT
<213>  Mus musculus

<400>  40

His Asp Glu Arg Ile Asp Tyr Ala Met Asp Tyr
1               5                   10

<210>  41
<211>  645

<212>  DNA
<213>  Mus musculus

<400>  41
gacatcaaga tgacccagtc tccatcttcc atgtatgcat ctctaggaga gagagtcact      60

atcacttgca aggcgagtca ggacattaat agctatttaa gctggttcca gcagaaacca     120

gggaaatctc ctaagaccct gatctatcgt gcaaacagat tggtagatgg ggtcccatca     180

aggttcagtg gcagtggatc tgggcaagat tattctctca ccatcagcag cctggagtat     240

gaagatatgg gaatttatta ttgtctacag tatgatgagt ttccgtatac gttcggatcg     300

gggaccaagc tggaaataaa acgaactgtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gttga                     645


<210>  42
<211>  107
<212>  PRT
<213>  Mus musculus

<400>  42

Asp Ile Lys Met Thr Gln Ser Pro Ser Ser Met Tyr Ala Ser Leu Gly
1               5                   10                  15


Glu Arg Val Thr Ile Thr Cys Lys Ala Ser Gln Asp Ile Asn Ser Tyr
            20                  25                  30


Leu Ser Trp Phe Gln Gln Lys Pro Gly Lys Ser Pro Lys Thr Leu Ile
        35                  40                  45


Tyr Arg Ala Asn Arg Leu Val Asp Gly Val Pro Ser Arg Phe Ser Gly
    50                  55                  60


Ser Gly Ser Gly Gln Asp Tyr Ser Leu Thr Ile Ser Ser Leu Glu Tyr
65                  70                  75                  80


Glu Asp Met Gly Ile Tyr Tyr Cys Leu Gln Tyr Asp Glu Phe Pro Tyr
                85                  90                  95


Thr Phe Gly Ser Gly Thr Lys Leu Glu Ile Lys
            100                 105


<210>  43

<211>  11
<212>  PRT
<213>  Mus musculus

<400>  43

Lys Ala Ser Gln Asp Ile Asn Ser Tyr Leu Ser
1               5                   10


<210>  44
<211>  7
<212>  PRT
<213>  Mus musculus

<400>  44

Arg Ala Asn Arg Leu Val Asp
1               5


<210>  45
<211>  9
<212>  PRT
<213>  Mus musculus

<400>  45

Leu Gln Tyr Asp Glu Phe Pro Tyr Thr
1               5


<210>  46
<211>  21
<212>  PRT
<213>  Unknown

<220>
<223>  Unknown

<400>  46

Met Arg Ser Leu Leu Ile Leu Val Leu Cys Phe Leu Pro Leu Ala Ala
1               5                   10                  15


Leu Gly Ala Ala Ala
            20


<210>  47
<211>  63
<212>  DNA
<213>  Unknown

<220>
<223>  Unknown

<400>  47
atgaggtctt tgctaatctt ggtgctttgc ttcctgcccc tggctgctct gggggcggcc      60

gcc                                                                    63

<210>  48
<211>  23
<212>  DNA
<213>  Artificial Sequence

<220>
<223>  Artificially synthesized primer sequence

<400>  48
cgactggagc acgaggacac tga                                               23


<210>  49
<211>  19
<212>  DNA
<213>  Artificial Sequence

<220>
<223>  Artificially synthesized primer sequence

<400>  49
aatttcttg tccacctgg                                                     19


<210>  50
<211>  26
<212>  DNA
<213>  Artificial Sequence

<220>
<223>  Artificially synthesized primer sequence

<400>  50
ctaacactca ttcctgttga agctct                                            26